# EUROPEAN PATENT SPECIFICATION

(11) **EP 4 003 507 B1**
(45) Date of publication and mention of the grant of the patent: **30.08.2023**
(21) Application number: 20771349.6
(22) Date of filing: 22.07.2020
(51) Int. Cl.: A61N 5/06, A61B 18/20, A61N 5/067, A61B 18/22, A61B 17/00, A61B 18/00

(54) **SYSTEM AND METHOD FOR GUIDED HAIR REMOVAL USING DIODE LASER**
SYSTEM UND VERFAHREN ZUR GEFÜHRTEN HAARENTFERNUNG UNTER VERWENDUNG EINES DIODENLASERS
SYSTÈME ET PROCÉDÉ D'ÉPILATION GUIDÉE À L'AIDE D'UN LASER À DIODE

(30) Priority: 24.07.2019 IT 201900012771
(43) Date of publication of application: 01.06.2022
(73) Proprietor: Mulè, Salvatore Vincenzo, 00054 Fiumicino (RM) (IT)
(72) Inventor: PERROTTA, Piero, 87027 Paola (CS) (IT)
(74) Representative: Giuliano, Natalia
(86) International application number: PCT/IB2020/056888
(87) International publication number: WO 2021/014375

(56) References cited:
- US-A1- 2007 198 004
- US-A1- 2013 103 017

## Description

The present invention relates to a system for guided hair removal using diode laser.

The present invention relates also to a method for guided hair removal using diode laser.

In particular, the present invention relates to a system for guided hair removal using diode laser, of the type that uses a two-stage device, i.e. a handpiece that can be used by an operator to administer the hair removal treatment, and a control unit connected to the handpiece by means of appropriate wiring.

In the existing systems for aesthetic treatments that use diode lasers, the handpiece includes, among its internal components, some items comprising Peltier modules, each of them having a cold side facing the patient subject to the treatment, and a warm side facing the inner side of the handpiece itself, a factor that involves heating the diode laser source, if the latter is mounted into the handpiece. Therefore, for such devices there is the need to cool the diode, typically using gas, air or more frequently liquid circuits, with a cooling liquid inlet conduit and an outlet conduit for the same liquid, which are enclosed in the wiring that connects the control unit to the handpiece. Consequently, the control unit must include a tank containing at least 40 cm³ of liquid that feeds the cooling circuit. The wiring between the control unit and the handpiece, in addition to the cooling liquid inlet and outlet pipes, also includes the electric wires that connect the diode to the control unit, and the electrical wires that connect the Peltier modules to the control unit, so increasing the section of the wiring itself, which can reach considerable dimensions.

Several systems for hair removal, achieved by applying a laser spot on different portions of the skin of a user, are currently known.

A first example Of known technical solution is described by the international application WO2015052645A1, whose subject-matter is a laser treatment apparatus comprising a main portable device able to be connected to an auxiliary attachment and having a laser source with a primary heat exchanger and a heat reduction circuit for supplying cooling fluid to the laser source. The cooling circuit, in addition, has a connection for an auxiliary circuit.

In addition, the patent application US2003032950A1 describes a photocosmetic device for use in medical or non-medical environments and that can be used for several tissue treatments, by means of a radiation that is sent to the tissue through optical systems designed to model said radiation and project it to a certain depth. The device has a variety of cooling systems, which include phase-changing solids and liquids to cool the treated skin and the radiation sources. Contact sensors and motion sensors can be used to improve the treatment. The device can also be modular to facilitate the maintenance and the replacement of parts.

Still, the patent application US2007198004A1 discloses an appliance to be used by a consumer in a non-medical environment. The device uses at least one source of low power electromagnetic radiation and can be positioned on an area to be treated for a prefixed time interval, or can be moved one or more times during each treatment. The appliance contains a radiation source control system, which may include several independently controlled sections.

The patent application US2013103017A1 describes a device for treating a portion of skin by scanning radiation to form a pattern of zones of the skin to be treated. The system includes an automated scanning system configured to receive an input beam generated by a radiation source and to scan the input beam generating a sequential series of output beams that form a pattern of zones of the skin to be treated. The automated scanning system includes a preferably cup-shaped element configured to rotate around an axis of rotation and that can include a plurality of lenses, each of them configured to provide one of the output beams.

Finally, the patent application US2013338662A1 describes methods, devices and systems for hair follicle treatments. One method may include the step of inserting a tissue dissection and modification (TDM) rod into an incision in a patient's skin. The TDM may comprise a tip having a plurality of protrusions with lysis segments positioned between the protrusions. The TDM may also include a window located on the top of the TDM itself, configured to supply energy to the hair follicles. After separating the tissue, using the lysis segments to define a target region, the window can be activated and moved within the target area to disable the hair follicles.

However, the known system for hair removal using diode laser suffer from limitations such as requiring a bulky cooling circuit, making the whole system uncomfortable for an operator who must carry out the treatment. In addition, during the hair removal treatment, the presence of the diode in the handpiece causes the heating of the same handpiece, due to the heat radiated from the hot side of the Peltier module, the adoption of which is necessary, since its cold side is suitable for cooling the skin of a user subject to the treatment.

Furthermore, the known systems for hair removal using laser sources have the further limitation that the power of the emitted laser beam and the wavelengths are fixed and set through the diode stack. Accordingly, to change the wavelengths it would be necessary to replace the entire handpiece.

A further limitation of the known systems is that they do not allow to provide a feedback about the quality of the treatments, nor do they provide feedback on the differences between subsequent treatments. An operator usually makes several passages of the handpiece on the areas of the skin to be treated, with the result of reducing the effectiveness of the treatment, at the same time exposing the patient to an excessive heat. In short, an operator sets the operating parameters of the laser hair removal system based on his personal experience, without the support of the control logic of the device.

The purpose of the present invention is to provide a system for guided hair removal using diode laser that allows a treatment of portions of the skin user-oriented, that is comfortable and efficient, having, therefore, characteristics such as to overcome the limitations that still affect the current systems for guided hair removal using diode laser, with reference to the known technique.

A further purpose of the present invention is to provide a system for guided hair removal using diode laser that provides continuous feedbacks, in real time, to an operator, allowing him to objectively evaluate the quality and the results of a treatment, avoiding to perform multiple passages on the portions of skin to be treated and to limit the discomfort for a user, especially regarding the exposure to heat.

According to the present invention, a system for guided hair removal using diode laser is provided, as defined in claim 1.

According to the present invention, a method for guided hair removal using diode laser is also provided, as defined in claim 9.

For a better understanding of the present invention, a preferred embodiment is now described, purely by way of nonlimiting example, with reference to the attached drawing, in which:
- figure 1 shows an overall view of a system for guided hair removal using diode laser, according to the invention;

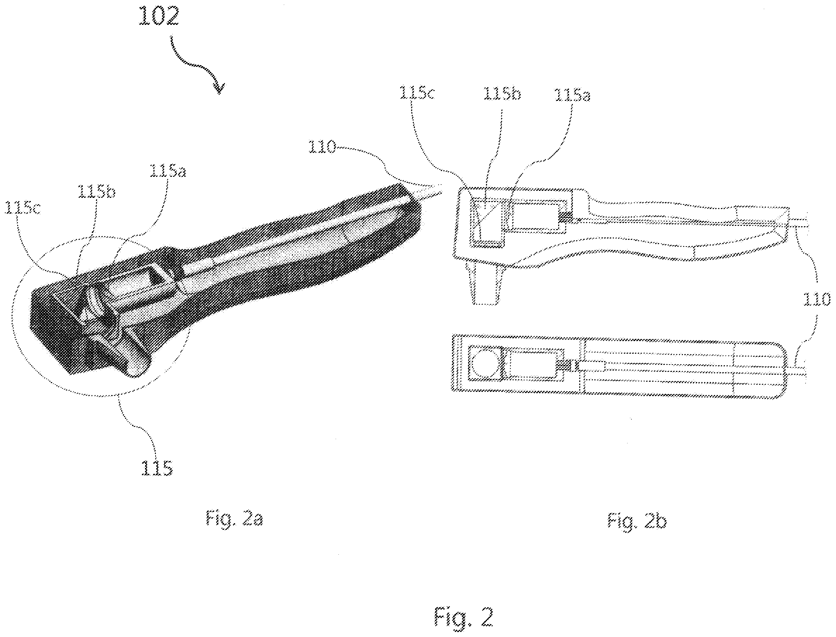

- figures 2a and 2b show a perspective and two sectional views, respectively, of a first component of the system for guided hair removal using diode laser, according to the invention;

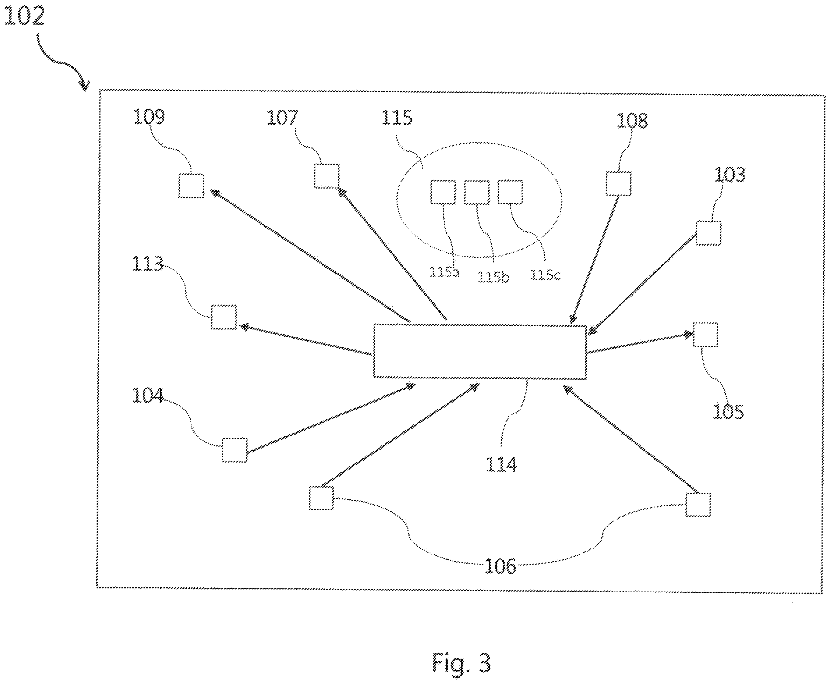

- figure 3 shows a block diagram of the first component of the system for guided hair removal using diode laser, according to the invention;

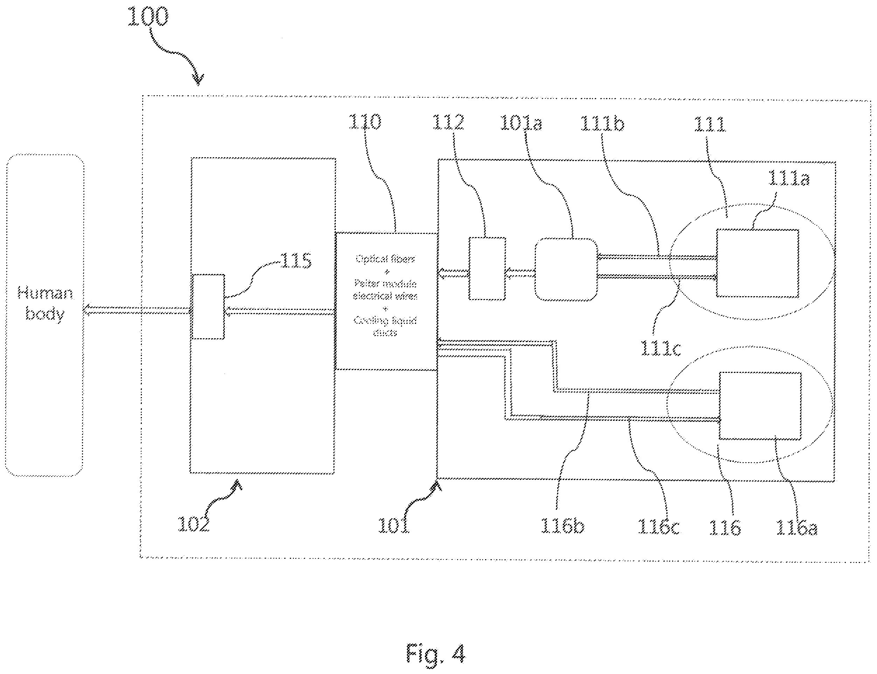

- figure 4 shows a block diagram of the system for guided hair removal using diode laser, according to the invention;
- figure 5 shows a block diagram of a portion of a second component of the system for guided hair removal using diode laser, according to the invention.

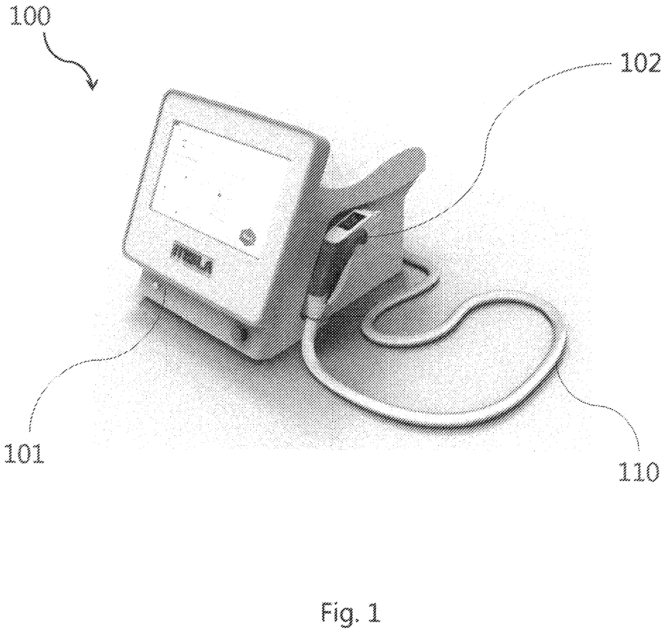

With reference to these figures and, in particular, to figure 1, a system for guided hair removal using diode laser is shown, according to the invention.

In particular, the system 100 for guided hair removal using diode laser comprises:
- a handpiece 102 configured to perform a laser hair removal treatment on a portion of the skin of a user, comprising: at least one position sensor 103; at least one temperature probe 104; at least one haptic transducer 105; at least one couple of pressure sensors 106; a camera 108 configured to detect in real time skin features and imperfections; an electronic control board 114 connected to the position sensor 103, to the temperature probe 104, to the haptic transducer 105, to the couple of pressure sensors 106, to the camera 108 e configured to receive signals from the position sensor 103 and from the camera 108, and to evaluate the spatial position, for example in a three-dimensional reference system, the displacement vector and the speed of the handpiece 102 during the laser hair removal treatment;
- a control unit 101 connected to the handpiece 102 through connection means 110 comprising optical fibers configured for the transmission of a laser spot from said control unit 101 to the handpiece 102.

According to an aspect of the invention, the handpiece 102 is separated from the control unit 101 and connected to said control unit 101 through the connection means 110.

According to an aspect of the invention, the handpiece 102 comprises, at one of its ends, an optical system 115 comprising a lens 115a, a prismatic mirror 115b coated with a dielectric material, and a sapphire glass window 115c.

According to an aspect of the invention, the control unit 101 comprises at least one laser diode 101a.

According to another aspect of the invention, the laser beam generated by the at least one laser diode 101a is conveyed by means of the optical fibers included in the connection means 110 that connect the control unit 101 to the handpiece 102.

Advantageously according to the invention, the presence of the optical system 115 included in the handpiece 102, and positioned at one end of the optical fibers allows to optimize the collimation of the laser beam whose spot is applied to a portion of the user's skin.

According to an aspect of the invention, the position sensor 103 is a MEMS device comprising a three-axis gyroscope and a three-axis accelerometer, and is identifiable as an IMU (Inertial Measurement Unit).

Advantageously according to the invention, the correlation of the signals generated by the position sensor 103 and the camera 108 allows to acquire in real time and continuously, by means of algorithms running on the electronic control board 114, the position and the components of the displacement vector of the handpiece 102, during a laser hair removal treatment.

According to an aspect of the invention, the camera 108 is configured to detect and evaluate in real time the characteristics of the skin surrounding the portion of said skin to be treated, for example the phototype, the number of hair, or skin imperfections such as skin spots and moles.

In use therefore, advantageously, it is possible to evaluate the variations of the skin phototype on each portion of skin to be treated, objectively evaluating the number of hair before and after the treatments, in order to estimate the performances achieved and the effectiveness.

Advantageously according to the invention, counting the number of hair before and after subsequent treatments, it is possible to detect information about the frequency and the number of treatments necessary to obtain a predetermined reduction in the number of hair.

According to an aspect of the invention, the at least one haptic transducer 105 is configured to signal specific working conditions of the handpiece 102 during a laser hair removal treatment.

Advantageously according to the invention, the haptic transducer 105 allows to warn the operator, through vibrational sequences, about erroneous actions of an operator who performs the treatment, for example an excessive speed of the handpiece 102 or a high number of passages of the same handpiece 102 on the portion of the skin to be treated, or even an incorrect positioning of the handpiece 102.

According to an aspect of the invention, the handpiece 102 comprises at least one loudspeaker 113, connected to the electronic control board 114, able to signal anomalies, which adds to the warnings generated by the haptic transducer 105, as well as signals that indicate the beginning and the end of the laser hair removal treatment.

According to an aspect of the invention, the handpiece 102 comprises a display 109 connected to the electronic control board 114 and configured for visualizing the graphical plot of the displacement vector and the position of the handpiece 102 during the execution of the laser hair removal treatment, performed by the operator.

Advantageously according to the invention, the display 109 allows to display information useful for the operator, for example the parameters set for the treatment and the variables acquired by the position sensor 103, i.e. the components of the displacement vector and the position of the handpiece 102, supporting the operator visually, providing an aid that is complementary to the tactile feedback provided by the haptic transducer 105.

According to an aspect of the invention, the couple of pressure sensors 106 is configured to activate a laser emission, referable as a laser spot, on the portion of the skin of the user to be treated, having an intensity directly proportional to the pressure level exercised by the operator on the handle of the handpiece 102.

Advantageously according to the invention, the couple of pressure sensors 106 allows to apply a laser spot having an adjustable intensity, such an application occurring only if an applied pressure is detected. In addition, the application of the laser spot is customized, depending on the specific operator carrying out the treatment, guided by the control unit 101 on the base the evaluation of the number of hair and the phototype, detected by means of the camera 108.

According to an aspect of the invention, the handpiece 102 comprises a Peltier module 107 connected to the electronic control board 114 and able to cool the portion of the user's skin to be treated.

Advantageously according to the invention, the handpiece 102 operates efficiently without generating a sort of thermal short circuit caused by the heat of the hot side of the Peltier module that, in known systems, adds to the heat produced by the laser diode when this is in use and placed into the handpiece.

According to another aspect of the invention, the connection means 110 comprise electrical connections of the Peltier module 107.

Advantageously according to the invention, the temperature probe 104 allows to detect continuously the temperature of the handpiece 102, in order to monitor potential overheating.

Advantageously according to the invention, thanks to the synergy of the camera 108, of the warnings of the haptic transducer 105, of the measures of the position sensor 103, of the information provided by the display 109 and of the sound warnings provided by the loudspeaker 113, the operator is able to administer in real-time the treatment effectively and comfortably for the user.

According to an aspect of the invention, the handpiece 102 has the shape of a parallelepiped, with a length preferably of 20 cm, a width preferably of 5 cm and a depth preferably of 6 cm.

According to another aspect of the invention, the display 109 has dimensions comprised between 2.0 inches and 2.4 inches, and preferably equal to 2.2 inches.

According to an aspect of the invention, the control unit 101 comprises a first cooling system 111 comprising at least one container 111 with deionized water having an output duct 111b to the at least one laser diode 101a and an inlet duct 111c from the at least one laser diode 101a.

Advantageously according to the invention, the first cooling system 111 includes shorter interconnections and a smaller filling tank, compared to those of known systems, in such a way as to increase the efficiency of the temperature control of the laser diode 101a.

According to another aspect of the invention, the control unit 101 comprises a further cooling system 116 comprising at least one container 116a with distilled water having an outlet duct 116b to the handpiece 102 and an inlet duct 116c from the handpiece 102.

According to another aspect of the invention, the connection means 110 comprise portions of the outlet duct 116b and of the inlet duct 116c.

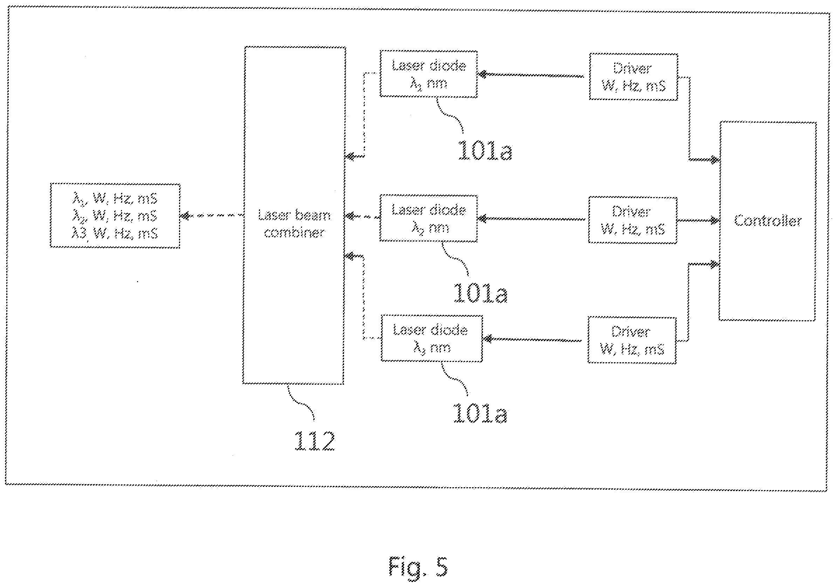

According to an aspect of the invention, the control unit 101 comprises an optical beam combiner 112, better shown in figure 5, configured for mixing and adjusting the laser beams having different wavelengths emitted by the at least one laser diode 101a, as shown in figure 5. In particular, the optical beam combiner 112 manages the "fast-axis collimation" and the "slow-axis collimation" of the laser beams emitted by the diode stack and their conveyance into the optical fibers. In this way, using specific electronic control boards, it is possible to modulate the power, pulses and emission times, making the treatments comfortable and effective.

According to another aspect of the invention, the control unit 101 allows to generate and transmit the laser beam to the handpiece 102 only in the absence of anomalies detected by the handpiece 102, for example an excessive temperature of the same handpiece 102 detected through the temperature probe 104, presence of moles, as previously said, detected by means of the camera 108, absence of pressure exercised on the handpiece 102 and detected by the couple of pressure sensors 106.

Advantageously according to the invention, the application of the laser spot that occurs only in the absence of the aforementioned anomalies, allows to ensure the integrity of the portion of the user's skin to be treated from potential further alterations.

Advantageously according to the invention, a firmware preloaded on the control unit 101 comprises a diagnostic module which includes, in addition to a self-diagnosis routine that is performed when the system 100 is started, a predictive fault logic aimed at optimizing the maintenance of the system 100.

Advantageously according to the invention, the electronic boards included in the control unit 101 are removable and replaceable with similar components, in order to minimize the shutdown of the system 100 for assistance.

According to an aspect of the invention, the system 100 is configured to send data related to a laser hair removal treatment to a database installed on at least one cloud server. In particular, such data are represented, for example, by the emission parameters of the laser beam, or by the frequency or effectiveness of the treatments to which the user is subject.

In use, therefore, advantageously, it is possible to create a personal record concerning any single user, with said user uniquely identifiable, for example by means of the tax code, through which to access the history of the laser hair removal sessions and verify the effectiveness of subsequent treatments. In this way, it is possible to optimize the parameters for future sessions, based on advanced historical data analyzes carried out using specific software installed on the cloud server.

Advantageously according to the invention, sharing data on remote databases installed on the cloud server allows to automatically set up customized hair removal treatment programs, based on optimized parameters depending on time series shared in the cloud.

According to another aspect of the invention, the system 100 is connected to a network, a local network or the Internet, through a wireless connection, WLAN o WWAN.

Another advantage of the system 100 according to the invention is that a potential variation of the wavelength set for a treatment does not require the replacement of the handpiece.

Another advantage of the diode laser guided hair removal system according to the invention is that, if a change in the size of the laser spot applied to the user's skin portion is required, there is no need to operate mechanically on the handpiece 102.

The present invention relates also to a method for guided hair removal using diode laser by means of the system 100, comprising the steps:
- of registering a user on a control unit of a system 100 for guided hair removal using diode laser;
- of initializing a session of a guided hair removal using diode laser for the user, registering a hair removal treatment for at least one portion of the skin of the user;
- of executing a pre-treatment by an operator comprising selecting a first portion of the skin to be treated, pointing the handpiece over said first portion of the skin, modifying settings of the system depending on images acquired through a camera included in the handpiece;
- of verifying by means of electronic boards included in the control unit first parameters extracted from the images acquired through the camera and able to start the next step;
- of executing a guided hair removal treatment by the operator over the first portion of the skin of the user depending on the first parameters and on second parameters such as the temperature of the handpiece, the presence of a mole, the pressure value of pressure sensors provided on opposite sides of the handpiece and able to activate, modulate and interrupt the treatment, said second parameters being able to be shown on a display on the handpiece and processed in real time by an electronic control board included in the handpiece and by the electronic boards included in the control unit, separated from the handpiece and connected to it through connection means, and on the basis of acoustic and haptic warnings generated by transducers included in the handpiece and connected to the electronic control board of the handpiece.

According to an aspect of the invention, during the step of registering a user, data related to the new user are provided and stored and a unique identification code is assigned to him. If, subsequently, even operating from another system 100, data already stored on a cloud server are entered, the system 100 will recall the historical data regarding the treatments already carried out by that specific user, in this way avoiding the operator to repeat the registration.

Advantageously according to the invention, the verification included in the step of verifying, by means of the electronic boards installed in the control unit, first parameters before the subsequent phase of carrying out a treatment, allows to fine-tune and customize the system 100 according to the specific needs of the user. The second parameters detected by the handpiece and sent to the control unit, according to which the step of executing a guided hair removal treatment takes place, are the temperature of the handpiece, the presence of a mole detected on the user's skin, the pressure value associated to the couple of pressure sensors. Subsequently, the electronic boards of the control unit analyze these parameters and send a pulse, i.e. a laser beam, to the handpiece only if the temperature is within a set range, no mole is detected, and pressure is detected on the handpiece. In such a case, a pulse generated by a stack of laser diodes mounted in the control unit is emitted, whose beam is conveyed in the direction of the handpiece through optical fibers included in the connection means. The aforementioned beam is variable and able to be modulated according to the first parameters, i.e., number of hair detected and phototype of the user. The previous actions are repeated cyclically, as the handpiece is moved to different portions of skin.

Advantageously according to the invention, the real-time evaluation of second parameters, for example the presence of moles or the high temperature of the handpiece, allows not to start an emission of a laser beam, with the simultaneous visualization of a warning message on the display of the handpiece, and to ensure the integrity of the portion of the user's skin.

Therefore, the system 100 according to the invention allows to administer to a user guided, optimized and comfortable aesthetic treatments, avoiding the operator to carry out an excessive number of handpiece passages on the skin portions to be treated.

Another advantage of the system for guided hair removal using diode laser according to the invention is that the handpiece, having a reduced number of hardware components, is lighter and smaller than that of known systems, allowing the operator to benefit from a remarkable handling during a laser hair removal treatment.

In this way, advantageously, positioning the at least one laser diode into the control unit increases the reliability of the handpiece, since this diode is a delicate component.

A further advantage of the system and the method for guided hair removal using diode laser is that they are safe, not allowing the application of the laser spot on the portion of the user's skin to be treated in the event of detection of moles, by means of the camera, on said portion of skin, simultaneously warning the operator and the user with a message shown on the display of the handpiece of the need for a specialist medical consultation.

Furthermore, the system for guided hair removal using diode laser according to the invention is efficient, not expensive and convenient to use.

Finally, the system and the method for guided hair removal using diode laser according to the invention allow to make the depilatory treatment comfortable for the user and easy to apply for the operator, and to make the treatment replicable on a personalized basis in different geographical locations, thanks to the sharing of the parameters of the user in the cloud.

The invention is defined in the following claims.

## Claims

1. System (100) for guided hair removal using diode laser comprising:
- a handpiece (102) configured to perform a laser hair removal treatment on a portion of the skin of a user comprising: at least one position sensor (103); at least one haptic transducer (105); at least one couple of pressure sensors (106); a camera (108) configured to detect in real time skin features and imperfections; a display (109); and an electronic control board (114) connected to the at least one position sensor (103), to the at least one haptic transducer (105), to the at least one couple of pressure sensors (106), to the camera (108), to the display (109) and configured to receive signals from the at least one position sensor (103) and from the camera (108), and to locate the spatial position, the displacement vector and the speed of the handpiece (102) during the laser hair removal treatment; and
- a control unit (101) comprising at least one laser diode (101a);
**characterized in that** the control unit (101) is connected to the handpiece (102) through connection means (110) comprising optical fibers configured for the transmission of a laser spot from said control unit (101) to the handpiece (102), said at least one couple of pressure sensors (106) being configured to activate a laser emission on the portion of the skin of the user to be treated, said laser emission having an intensity directly proportional to the pressure level exercised by an operator, and said display (109) being configured to visualize the graphical plot of the displacement vector and the position of the handpiece (102) during the execution of the laser hair removal treatment.

2. System (100) according to claim 1, wherein the at least one position sensor (103) is a MEMS device comprising a three-axis gyroscope and a three-axis accelerometer.

3. System (100) according to claim 1, wherein the at least one haptic transducer (105) is configured to signal specific working conditions of the handpiece (102) during a laser hair removal treatment.

4. System (100) according to claim 1, wherein the handpiece (102) comprises a Peltier module (107) connected to the electronic control board (114) and able to cool the portion of the skin of the user to be treated, and in that the connection means (110) comprise electrical connections of the Peltier module (107).

5. System (100) according to claim 1, wherein the handpiece (102) comprises at least one loudspeaker (113) connected to the electronic control board (114) and able to signal anomalies, warnings, and signals indicating the beginning and the end of the laser hair removal treatment.

6. System (100) according to claim 1, wherein the control unit (101) comprises a first cooling system (111) comprising at least one container (111a) with deionized water having an output duct (111b) to the at least one laser diode (101a) and an inlet duct (111c) from the at least one laser diode (101a), and a further cooling system (116) comprising at least one container (116a) with distilled water having an output duct (116b) to the handpiece (102) and an inlet duct (116c) from the handpiece (102).

7. System (100) according to claim 1, wherein the control unit (101) comprises an optical beam combiner (112) configured for mixing and adjusting the laser beams having different wavelengths emitted by the at least one laser diode (101a).

8. System (100) according to claim 1, being configured to send data related to a guided hair removal treatment using diode laser to a database installed on at least one cloud server.

9. Method for guided hair removal using diode laser by means of the system (100) according to one of the previous claims, comprising the steps:
- of registering a user on a control unit of a system for guided hair removal using diode laser;
- of initializing a session of a guided hair removal using diode laser for the user registering a hair removal treatment for at least one portion of the skin of the user;
- of executing a pre-treatment by an operator comprising selecting a first portion of the skin to be treated, pointing the handpiece over said first portion of the skin, modifying settings of the system depending on images acquired through a camera included in the handpiece;
- of verifying by means of electronic boards included in the control unit first parameters extracted from the images acquired through the camera and able to start the next step;
- of executing a guided hair removal treatment by the operator over the portion of the skin of the user depending on the first parameters and on second parameters able to be visualized by means of a display included in the handpiece, said second parameters being processed in real time by an electronic control board included in the handpiece and by the electronic boards include in the control unit separated from the handpiece and connected to it through connections means, and depending on acoustic and haptic warnings generated by transducers included in the handpiece and connected to the electronic control board of the handpiece.

## Patentansprüche

1. System (100) zur geführten Haarentfernung mittels Diodenlaser, umfassend:
- ein Handstück (102), das so konfiguriert ist, dass es eine Laser-Haarentfernungsbehandlung an einem Teil der Haut eines Benutzers durchführt, umfassend: mindestens einen Positionssensor (103); mindestens einen haptischen Wandler (105); mindestens ein Paar von Drucksensoren (106); eine Kamera (108) eine Kamera (108), die so konfiguriert ist, dass sie in Echtzeit Hautmerkmale und Unvollkommenheiten erkennt; eine Anzeige (109); und eine elektronische Steuerplatine (114), die mit dem mindestens einen Positionssensor (103), dem mindestens einen haptischen Wandler (105), dem mindestens einen Paar Drucksensoren (106), der Kamera (108) und der Anzeige (109) verbunden ist und so konfiguriert ist, dass sie Signale von dem mindestens einen Positionssensor (103) und von der Kamera (108) empfängt, und um die räumliche Position, den Verschiebungsvektor und die Geschwindigkeit des Handstücks (102) während der Laserhaarentfernungsbehandlung zu lokalisieren; und
- eine Steuereinheit (101) mit mindestens einer Laserdiode (101a);
**dadurch gekennzeichnet, dass** die Steuereinheit (101) mit dem Handstück (102) über Verbindungsmittel (110) verbunden ist, die optische Fasern umfassen, die für die Übertragung eines Laserpunkts von der Steuereinheit (101) zum Handstück (102) konfiguriert sind, wobei das mindestens eine Paar von Drucksensoren (106) konfiguriert ist, um eine Laseremission auf dem Teil der Haut des zu behandelnden Benutzers zu aktivieren, wobei die Laseremission eine Intensität aufweist, die direkt proportional zu dem von einem Bediener ausgeübten Druckniveau ist, und wobei die Anzeige (109) so konfiguriert ist, dass sie die grafische Darstellung des Verschiebungsvektors und der Position des Handstücks (102) während der Ausführung der Laserhaarentfernungsbehandlung visualisiert.

2. System (100) nach Anspruch 1, wobei mindestens eine Positionssensor (103) eine MEMS-Vorrichtung ist, die ein Drei-Achsen-Gyroskop und einen Drei-Achsen-Beschleunigungsmesser umfasst.

3. System (100) nach Anspruch 1, wobei der mindestens eine haptische Wandler (105) so konfiguriert ist, dass er spezifische Arbeitsbedingungen des Handstücks (102) während einer Laserhaarentfernungsbehandlung signalisiert.

4. System (100) nach Anspruch 1, wobei das Handstück (102) ein Peltier-Modul (107) umfasst, das mit der elektronischen Steuerplatine (114) verbunden und in der Lage ist, den zu behandelnden Teil der Haut des Benutzers zu kühlen, und dass die Verbindungsmittel (110) elektrische Verbindungen des Peltier-Moduls (107) umfassen.

5. System (100) nach Anspruch 1, wobei das Handstück (102) mindestens einen Lautsprecher (113) umfasst, der mit der elektronischen Steuerplatine (114) verbunden und in der Lage ist, Anomalien, Warnungen und Signale zu vermitteln, die den Beginn und das Ende der Behandlung der Laserhaarentfernung anzeigen.

6. System (100) nach Anspruch 1, wobei die Steuereinheit (101) ein erstes Kühlsystem (111) umfasst, das mindestens einen Behälter (111a) mit entionisiertem Wasser mit einem Ausgangskanal (111b) zu der mindestens einen Laserdiode (101a) und einem Eingangskanal (111c) von der mindestens einen Laserdiode (101a) umfasst, und ein weiteres Kühlsystem (116), das mindestens einen Behälter (116a) mit destilliertem Wasser umfasst, der einen Ausgangskanal (116b) zu dem Handstück (102) und einen Eingangskanal (116c) von dem Handstück (102) hat.

7. System (100) nach Anspruch 1, wobei die Steuereinheit (101) einen optischen Strahlkombinierer (112) umfasst, der so konfiguriert ist, dass er die von der mindestens einen Laserdiode (101a) emittierten Laserstrahlen mit unterschiedlichen Wellenlängen mischt und einstellt.

8. System (100) nach Anspruch 1, das so konfiguriert ist, dass es Daten in Bezug auf eine geführten Haarentfernungsbehandlung mit Diodenlaser an eine Datenbank zu senden, die auf mindestens einem Cloud-Server installiert ist.

9. Verfahren zur geführten Haarentfernung mit Diodenlaser mittels des Systems (100) nach einem der vorhergehenden Ansprüche, umfassend die Schritte:
- der Registrierung eines Benutzers einer Steuereinheit eines Systems zur geführten Haarentfernung mit Diodenlaser;
- der Initialisierung einer Sitzung einer geführten Haarentfernung mit Diodenlaser für den Benutzer, die eine Haarentfernungsbehandlung für mindestens einen Teil der Haut des Benutzers registriert;
- der Durchführung einer Vorbehandlung durch einen Bediener, die das Auswählen eines ersten zu behandelnden Hautabschnitts, das Richten des Handstücks auf den ersten Hautabschnitt und das Ändern der Einstellungen des Systems in Abhängigkeit von den durch eine im Handstück enthaltene Kamera erfassten Bildern umfasst;
- mit Hilfe von elektronischen Platinen, die in der Steuereinheit enthalten sind, erste Parameter zu überprüfen, die aus den von der Kamera aufgenommenen Bildern extrahiert wurden, um den nächsten Schritt zu starten;
- Durchführung einer geführten Haarentfernungsbehandlung durch den Bediener auf dem Hautabschnitt des Benutzers in Abhängigkeit von den ersten Parametern und von zweiten Parametern, die mittels eines in dem Handstück enthaltenen Displays sichtbar gemacht werden können, wobei die zweiten Parameter in Echtzeit durch eine in dem Handstück enthaltene elektronische Steuerplatine und durch die elektronischen Platinen verarbeitet werden, die in der von dem Handstück getrennten und mit diesem durch Verbindungsmittel verbundenen Steuereinheit enthalten sind, und in Abhängigkeit von akustischen und haptischen Warnungen, die durch in dem Handstück enthaltene und mit der elektronischen Steuerplatine des Handstücks verbundene Wandler erzeugt werden.

## Revendications

1. Système (100) d'épilation guidée avec laser à diode comprenant :
- une pièce à main (102) configurée pour effectuer un traitement d'épilation au laser sur une partie de peau d'un utilisateur et comprenant : au moins un capteur de position (103) ; au moins un transducteur haptique (105) ; au moins un couple de capteurs de pression (106) ; une caméra (108) configurée pour détecter en temps réel les caractéristiques et les imperfections de la peau ; un écran (109) ; et une carte de commande électronique (114) connectée à au moins un capteur de position (103), à au moins un transducteur haptique (105), à au moins un couple de capteurs de pression (106), à la caméra (108), à l'écran (109) et configurée pour recevoir des signaux d'au moins un capteur de position (103) et de la caméra (108), et pour localiser la position dans l'espace, le vecteur de déplacement et la vitesse de la pièce à main (102) pendant le traitement d'épilation au laser ; et
- une unité de commande (101) comprenant au moins une diode laser (101a) ;
le système étant **caractérisé par** le raccordement de l'unité de commande (101) à la pièce à main (102) par des moyens de connexion (110) comprenant des fibres optiques configurées pour la transmission d'un spot laser de l'unité de commande (101) susmentionnée à la pièce à main (102) ; le couple susmentionné, au moins un, de capteurs de pression (106) étant configuré pour activer une émission laser sur la partie de peau à traiter de l'utilisateur, l'émission laser ayant une intensité directement proportionnelle au niveau de pression exercé par un opérateur ; et l'écran sus-indiqué(109) étant configuré pour visualiser le tracé graphique du vecteur de déplacement et la position de la pièce à main (102) pendant l'exécution du traitement d'épilation au laser.

2. Système (100) selon la revendication 1, dans lequel le capteur de position (103), au nombre d'un au minimum, est un dispositif MEMS comprenant un gyroscope à trois axes et un accéléromètre à trois axes.

3. Système (100) selon la revendication 1, dans lequel au moins un transducteur haptique (105) est configuré pour signaler des conditions de travail spécifiques de la pièce à main (102) pendant un traitement d'épilation au laser.

4. Système (100) selon la revendication 1, dans lequel la pièce à main (102) comprend un module Peltier (107) raccordé à la carte de commande électronique (114) et capable de refroidir la partie de peau à traiter de l'utilisateur, et dans lequel les moyens de connexion (110) comprennent les raccords électriques du module Peltier (107).

5. Système (100) selon la revendication 1, dans lequel la pièce à main (102) comprend au moins un haut-parleur (113) relié à la carte de commande électronique (114) et en mesure de signaler des anomalies, des avertissements et des signaux indiquant le début et la fin du traitement d'épilation au laser.

6. Système (100) selon la revendication 1, dans lequel l'unité de commande (101) comprend : un premier système de refroidissement (111) avec au moins un récipient (111a) contenant de l'eau déionisée et doté d'un conduit de sortie (111b) vers un nombre minimum d'une diode laser (101a), et d'un nombre minimum d'un conduit d'entrée (111c) avec au moins une diode laser (101a) ; et un autre système de refroidissement (116) avec au moins un récipient (116a) contenant de l'eau distillée et doté d'un conduit de sortie (116b) vers la pièce à main (102) et d'un conduit d'entrée (116c) depuis la pièce à main (102).

7. Système (100) selon la revendication 1, dans lequel l'unité de commande (101) comprend un mélangeur de faisceaux optiques (112) configuré de sorte à mélanger et ajuster des faisceaux laser ayant différentes longueurs d'onde émises par au moins une diode laser (101a).

8. Système (100) selon la revendication 1, configuré pour envoyer à une base de données installée sur au moins un serveur en nuage des données relatives à un traitement d'épilation guidée à l'aide d'un laser à diode.

9. Procédé d'épilation guidée au moyen d'un laser à diode au moyen du système (100) selon l'une des revendications précédentes, comprenant les étapes suivantes :
- enregistrement d'un utilisateur sur une unité de commande d'un système d'épilation guidée par laser à diode ;
- initialisation d'une séance d'épilation guidée au laser diode pour l'utilisateur enregistrant un traitement d'épilation pour au moins une partie de peau de l'utilisateur ;
- exécution d'un prétraitement par un opérateur comprenant la sélection d'une première partie de peau à traiter, orientation de la pièce à main sur ladite première partie de peau, modification des paramètres du système en fonction des images acquises par une caméra incluse dans la pièce à main ;
- vérification, au moyen de cartes électroniques incluses dans l'unité de commande, des premiers paramètres extraits des images acquises par la caméra et capables de lancer l'étape suivante ;
- exécution d'un traitement d'épilation guidé par l'opérateur sur la partie de peau de l'utilisateur : en fonction des premiers et des seconds paramètres pouvant être visualisés au moyen d'un écran inclus dans la pièce à main, les seconds paramètres étant traités en temps réel par une carte de commande électronique incluse dans la pièce à main et par les cartes électroniques incluses dans l'unité de commande séparée de la pièce à main et reliée à celle-ci par des moyens de raccord ; et en fonction d'avertissements acoustiques et haptiques générés par des transducteurs inclus dans la pièce à main et reliés à la carte de commande électronique de la pièce à main.
